(19) Europäisches Patentamt
European Patent Office
Office européen des brevets

(11) Publication number: 0 033 042 B1

(12) EUROPEAN PATENT SPECIFICATION

(45) Date of publication of patent specification: 22.08.84

(51) Int. Cl.$^3$: A 61 K 9/06, A 61 K 31/18, A 61 K 31/41

(21) Application number: 80400129.5

(22) Date of filing: 28.01.80

# (54) Ophthalmic inserts for lowering intraocular pressure comprising carbonic anhydrase inhibitors.

(43) Date of publication of application:
05.08.81 Bulletin 81/31

(45) Publication of the grant of the patent:
22.08.84 Bulletin 84/34

(84) Designated Contracting States:
AT BE CH DE FR GB IT LU NL SE

(56) References cited:
FR-A-2 332 008
GB-A- 851 287
GB-A- 860 371
US-A-4 008 719

CHEMICAL ABSTRACTS, vol. 89, no. 5, July 31, 1978 page 46, ref.: 36692d Columbus Ohio, US, R. KIMURA: "Effect of long-term subconjunctival administration of Diamox (acetazolamide) on the ocular tension in rabbit"

CHEMICAL ABSTRACTS, vol. 88, no. 25, June 19, 1978, page 147, ref.: 183569b Columbus, Ohio, US, M. TAKASE: "Effects of acetazolamide and epinephrine on the aqueous flow rate in the rhesus monkey"

(73) Proprietor: MERCK & CO. INC.
126, East Lincoln Avenue P.O. Box 2000
Rahway New Jersey 07065 (US)

(72) Inventor: Smith, Robert L.
1355 Pickwick Lane
Lansdale, Pennsylvania, 19446 (US)

(74) Representative: Corre, Jacques Denis Paul et al
Cabinet Regimbeau 26, Avenue Kléber
F-75116 Paris (FR)

(56) References cited:
Carlo Runti: "FONDAMENTI DI CHIMICA FARMACEUTICA", V1, 1971, Editions Lint Trieste IT

A.M.A. Archives of Ophthalmology, Vol. 51 (6), June 54, pp. 735-739

Am J. Ophthalmol. (39), pp. 336-339 (1955)

Am J. Ophthalmol (47) pp. 342-361 (1959)

Hugh Davson "The Eye", Academic Press, New York-London (1962), Vol. 1, pp. 133, 138, 141

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).



Courier Press, Leamington Spa, England.

(56) References cited:
R.P. Thomas, J. Ophthalm. 60 (1965) pp. 241, 243, 245

W.H. Havener "Ocular Pharmacology", C.V. Mosby Co., St. Louis (1966) pp. 396-418

"Symposium on Ocular Pharmacology and Therapeutics", Transactions of the New Orleans Academy of Ophthalmology, The C.V. Mosby Co., St. Louis, (1970) pp. 132-136

Investigative Ophthalmology, Vol. 13 (7), July 1974, pp. 479-483

**Description**

This invention relates to ophthalmic inserts for lowering intraocular pressure comprising carbonic anhydrase inhibitors that are topically effective in the treatment of elevated intraocular pressure. More particularly, it relates to inserts comprising the alkali metal salts of salt-forming carbonic anhydrase inhibitors that, when applied topically to the eye, are transported to the cilary process. In particular, this invention relates to inserts comprising the sodium and potassium salts of salt-forming carboxy anhydrase inhibitors.

Glaucoma is a degenerative disease of the eye wherein the pressure in the eye (i.e., intraocular pressure) is too high for the normal function of the eye and, as a result, damage occurs to the optic nerve head and results in irreversible loss of visual function. If untreated, glaucoma will eventually lead to blindness. Ocular hypertension, i.e., the condition of elevated intraocular pressure without optic nerve head damage or characteristic glaucomatous visual field loss, is now believed by the majority of ophthalmologists to merely represent the earliest phase in the onset of a glaucoma.

A number of the drugs presently employed to treat glaucoma are not entirely satisfactory, particularly in the earliest course of the disease when the side effects they produce are often worse than the symptoms of the disease.

Pilocarpine, for example, although systemically harmless and quite effective, causes considerable local difficulties. The pupil constricts so that little light becomes available to the eye and the eye loses its ability to adapt from light to dark. Accommodation is stimulated so that the patient's refraction is sometimes incorrect and vision is blurred. The drug itself causes a local vasodilitation and red eyes and irritation are common. In short, although valuable, it really is unsatisfactory as a first line drug.

When carbonic anhydrase inhibitors are used systemically they have a number of disadvantages. While extremely effective in lowering intraocular pressure, they often cause a numbness and tingling, gastrointestinal upsets and, frequently, depression, lethargy, and a loss of appetite, and general malaise. These, in addition to the occasional more severe systemic complications such as aplastic anemia, are so common that many physicians are reluctant to routinely prescribe carboxy anhydrase inhibitors. As a consequence, only highly motivated patients who understand the seriousness of their condition will faithfully continue medication.

While investigators have long realized the benefits that would accompany topical administration, the selection of the proper entity has long eluded them. Reports in the literature indicate that carbonic anhydrase inhibitors are inactive topically. Even the extreme measure of direct injection into the anterior chamber is reported to have no pressure lowering effect; see for example:

W. M. Grant and R. R. Trotter, *Arch. Ophthalmol., 51*, 735 (1954).

R. H. Foss, *Am. J. Ophthalmol., 39*, 336 (1955).

B. Becker, *Am. J. Ophthalmol., 47* 342 (1959).

H. Davson, "The Eye", Vol. I, Academic Press, N.Y., 1962, p. 133.

R. P. Thomas and M. W. Riley, *Am. J. Ophthalmol., 60*, 241 (1965).

W. H. Havener, "Ocular Pharmacology", C. V. Mosby Co., St. Louis, 1966, pp. 395—419.

S. M. Drance, Chapter 13 in "Symposium on Ocular Pharmacology and Therapeutics", C. V. Mosby Co., St. Louis, 1970, pp. 132—142.

T. H. Maren, *Invest. Ophthalmol., 13,* 479 (1974).

In the face of this collection of negative findings, it is now discovered that if an alkali metal salt, most preferably the sodium or potassium salt, of a carbonic anhydrase inhibitor is employed as a topical agent, the pressure lowering effects are substantially equivalent to systemic administration of the parent drug.

Because carbonic anhydrase inhibitors have a profound effect in altering basic metabolism, the avoidance of a systemic route serves to diminish, if not entirely eliminate, those side effects caused by metabolic acidosis such as vomiting, numbness, tingling, and general malaise.

Thus the invention relates to an ophthalmic insert for lowering intraocular pressure characterized in that it comprises a mixture of at least:

—a pressure reducing amount of an alkali metal salt of a carbonic anhydrase inhibitor selected among 4,5 - dichloro - m - benzene - disulfonamide; N - [5 - (aminosulfonyl) - 3 - methyl - 1,3,4 - thiadiazol - 2(3H) - ylidene] - acetamide; N - [5 - (aminosulfonyl) - 3 - methyl - 1,3,4 - thiadiazol - 2(3H) - ylidene] - acetamide; p - sulfamoylbenzoic acid; N - [5 - (aminosulfonyl) - 1,3,4 - thiadiazol - 2 - yl]propanamide; N - [5 - (aminosulfonyl) - 1,3,4 - thiadiazol - 2 - yl]butanamide; and 5 - benzenesulfonamido - 1,3,4 - thiadiazol - 2 - sulfonamide; and

—an ophthalmologically acceptable water soluble polymer;

the polymer being shaped under the form of a solid insert suitable to be put in the eye.

In the practice of this invention, the alkali metal salts of carbonic anhydrase inhibitor are prepared from the known, salt-forming carbonic anhydrase inhibitors, the following of which are preferred:

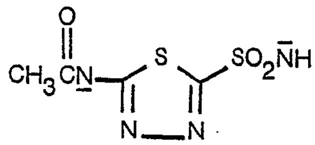

· $2\overset{+}{M}$ ;

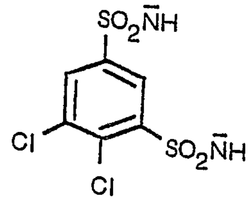

· $2\overset{+}{M}$ ;

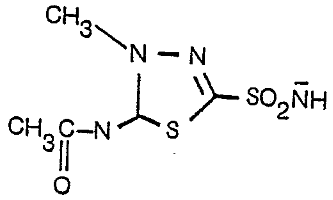

· $2\overset{+}{M}$ ; and

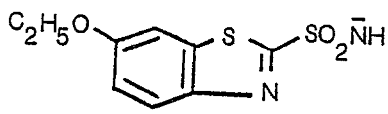

· $2\overset{+}{M}$

where $\overset{+}{M}$ is an alkali metal cation. As used herein, the term "alkali metal" includes lithium, sodium, potassium, cesium and rubidium.

These salts may be hygroscopic and, as a consequence, may occur as hydrated species. When formulating into the inserts, the material should be kept as dry as possible during manufacture and protected from excessive moisture during storage and before use. The preferred alkali metal salts include as cations, potassium, sodium, and rubidium, with the most preferred being potasium and sodium. In inserts, the potassium and sodium forms are both preferred cations.

Other carbonic anhydrase inhibitors can be used if they are capable of forming an alkali metal salt. Especially useful are those carbonic anhydrase inhibitors which have the sulfamoyl moiety (i.e., $-SO_2NH_2$) as a substituent. This can form the salt, $-SO_2\overset{-}{N}H\overset{+}{M}$, where $\overset{+}{M}$ is defined as above. Thus, carbonic anhydrase inhibitors such as *p*-sulfamoylbenzoic acid; N-[5-(aminosulfonyl)-1,3,4-thiadiazol-2-yl]propanamide; N-[5-(aminosulfonyl)-1,3,4-thiadiazol-2-yl]butanamide; and 5-benzenesulfonamido-1,3,4-thiadiazol-2-sulfonamide can also be used when formulating the carbonic anhydrase inhibitor alkali metal salts into an ophthalmic preparation, e.g., from 0.1% to 5% by weight can be employed. The objective is to administer a dose of from 0.1 to 10 mg per eye to the patient.

The compounds of this invention are administered in the form of solid inserts. Formulations of these compounds may contain from 0.01 to 5% and especially 0.5 to 2% of medicament. Higher dosages as, for example, about 10%, or lower dosages can be employed provided the dose is effective in lowering intraocular pressure. As a unit dosage from between 0.001 to 10.0 mg, preferably 0.005 to 2.0 mg, and especially 0.1 to 1.0 mg of the compound is generally applied to the human eye, generally on a daily basis.

The pharmaceutical preparation according to the invention are in the form of a solid insert. For example, one may use a solid water soluble polymer as the carrier for the medicament. Inserts that are known in the art that are suitable for use with this include those set forth and described in U.S.P. 3,993,071; U.S.P. 3,986,510; U.S.P. 3,868,445; and U.S.P. 3,867,510 employing the formulation and fabrication techniques described therein. The polymer used to form the insert may be any water soluble non-toxic polymer, for example, cellulose derivatives such as methyl cellulose, alkali metal carboxymethyl cellulose, hydroxyethyl cellulose, hydroxypropyl cellulose, hydroxypropylmethyl cellulose; acrylates such as polyacrylic acid salts, ethylacrylates; polyacrylamides; natural products such as gelatin, alginates, pectins, tragacanth, karaya, chondrus, agar, acacia; the starch derivatives such as starch acetate, hydroxyethyl starch ethers, hydroxypropyl starch, as well as other synthetic derivatives such as polyvinyl alcohol, polyvinyl pyrrolidone, polyvinyl methyl ether, polyethylene oxide, neutralized carbopol and xanthan gum and mixtures of said polymer.

The solid insert according to the invention is preferably prepared from cellulose derivatives such as methyl cellulose, hydroxyethyl cellulose, hydroxypropyl cellulose of hydroxypropylmethyl cellulose or from other synthetic materials such as polyvinyl alcohol, polyvinyl pyrrolidone, polyethylene oxide or polyvinyl methylether.

Hydroxypropyl cellulose, one of the preferred polymers for the preparation of the insert is available in several polymeric forms, all of which are suitable in the preparation of these inserts. Thus, the product sold by Hercules, Inc. of Wilmington, Delaware, under the name KLUCEL® such as KLUCEL® HF, HWF, MF, GF, JF, LF and EF, which are intended for food or pharmaceutical use, are particularly useful. The molecular weight of these polymers useful for the purposes described herein may be at least 30,000 to about 1,000,000 or more.

Similarly, an ethylene oxide polymer having a molecular weight of up to 5,000,000 or greater, and preferably 100,000 to 5,000,000 can be employed.

Further, for example, POLYOX®, a polymer supplied by Union Carbide Co., may be used having a molecular weight of about 50,000 to 5,000,000 or more and preferably 3,000,000 to 4,000,000. Other specific polymers which are useful are polyvinyl pyrrolidine having a molecular weight of from about 10,000 to about 1,000,000 or more, preferably up to about 350,000 and especially about 20,000 to 60,000; polyvinyl alcohol having a molecular weight of from about 30,000 to 1,000,000 or more, particularly about 400,000 and especially from about 100,000 to about 200,000; hydroxypropylmethyl cellulose having a molecular weight of from about 10,000 to 1,000,000 or more, particularly up to about 200,000 and especially about 80,000 to about 125,000; methyl cellulose having a molecular weight of from about 10,000 to about 1,000,000 or more, preferably up to about 200,000 and especially about 50 to 100,000; and CARBOPOL® (carboxyvinyl polymer) of B. F. Goodrich and Co. designated as grades 934, 940 and 941.

For the purpose of this invention the type and molecular weight of the polymer is not critical. Any water soluble polymers can be used which have an average molecular weight which will afford dissolution of the polymer and, accordingly, the medicament in any desired length of time. The inserts, therefore, can be prepared to allow for retention and, accordingly, effectiveness in the eye for any desired period. The insert can be in the form of a square, rectangle, oval, circle, doughnut, semi-circle, 1/4 moon shape, and the like. Preferably, the insert is in the form of a rod, doughnut, oval or 1/4 moon. The insert can be readily prepared, for example, by dissolving the medicament and the polymer in a suitable solvent and evaporating the resulting solution to afford a thin film of the polymer which can then be subdivided to prepare inserts of appropriate size. Alternatively, the insert can be prepared by intimately admixing polymer and the medicament and thereafter molding the resulting mixture under the influence of heat and pressure to form a thin film. Preferably, the inserts are prepared by molding or extrusion procedures well known in the art. The molded or extruded product can then be subdivided to afford inserts of suitable size for administration in the eye.

The insert can be of any suitable size which readily fits into the eye. For example, castings or compression molded films having a thickness of about 0.25 mm to 1.50 mm can be subdivided to obtain suitable inserts. Rectangular segments of the cast or compressed film having a thickness between about 0.5 and 1.5 mm can be cut to afford shapes such as rectangular plates of 4 × 5—20 mm or ovals of comparable size. Similarly, extruded rods having a diameter between about 0.5 and 1.5 mm can be cut into suitable sections to provide the desired amount of polymer. For example, rods of 1.0 to 1.5 mm in diameter and about 2—20 mm long are found to be satisfactory. The inserts may also be directly formed by injection molding. It is preferred that the ophthalmic inserts containing the medicament of the present invention be formed so that they are smooth and do not have any sharp edges or corners which could cause damage to the eye. Since the term smooth and sharp edges or corners are subjective terms, in this application these terms are used to indicate that excessive irritation of the eye will not result from the use of the insert.

The medicated ocular inserts can also contain plasticizers, buffering agents, appropriate inert fillers, and preservatives. Plasticizers suitable for this purpose must, of course, also be completely soluble in the lacrimal fluids of the eye. Examples of suitable plasticizers that might be mentioned are water, polyethylene glycol, propylene glycol, glycerine, trimethylol propane, di- and tripropylene glycol and hydroxypropyl sucrose. Typically, such plasticizers can be present in the medicated ophthalmic insert in an amount ranging from up to 1 to about 30% by weight. A particularly preferred plasticizer is water which is present in amounts of at least about 5% up to about 40%. In actual practice, a water content of from about 10% to about 20% is preferred since it may be easily accomplished and adds the desired softness and pliability to the insert.

When plasticizing the solid medicinal product with water, the product is contacted with air having a relative humidity of at least 40% until said product picks up at least about 5% water and becomes softer and more pliable. In a preferred embodiment, the relative humidity of the air is from about 60% to about 99% and the contact is continued until the water is present in the product in amounts of from about 10% to about 20%.

Suitable water soluble preservatives which may be employed in the insert are alkali bisulfate, alkali thiosulfate, ascorbate, benzalkonium chloride, chlorobutanol, thimerosal, phenylmercuric acetate, phenylmercuric borate, parabens, benzyl alcohol and phenyl ethanol. These agents may be present in amounts of from 0.001 to 5% by weight of solid insert, and preferably 0.1 to 2%.

Suitable water soluble buffering agents are alkali, alkali earth carbonates, phosphates, bicarbonates, citrates, borates, and the like, such as sodium or potassium phosphate, citrate, borate, acetate, bicarbonate and carbonate. These agents may be present in amounts sufficient to obtain a pH of the system of between 5.5 to 8.1 and especially 7—8; usually up to about 2% by weight of polymer.

The insert may contain from about 1 mg to 100 mg of water soluble polymer, more particularly from 5 to 50 mg and especially from 5 to 20 mg. The medicament is present from about 0.1 to about 25% by weight of insert.

The following examples serve to more fully illustrate this invention.

Ophthalmic inserts are manufactured from a solvent cast film prepared by making a viscous solution of the powder blend using a methanol/water solvent system (10 ml methanol is added to 2.5 g of powder blend, to which 11 ml of water (in three divided portions) is added). The solution is placed on a Teflon® polytetrafluoroethylene plate and allowed to dry at ambient conditions. After drying, the film is placed in an 88% relative humidity cabinet until it is pliable. Appropriately sized inserts are then cut from the film.

Example 1

$Na^+$ salt of methazolamide 1 mg
Hydroxypropyl cellulose q.s. a.d. 12 mg

Ophthalmic inserts are manufactured from compression molded films which are prepared on a Carver Press by subjecting the powder mixture of the above ingredients to a compressional force of 82.8 MPa (12000 psi) (gauge) at 177°C for one minute. The film is cooled under pressure by having cold water circulate in the platen. Ophthalmic inserts are then individually cut from the film with a punch. Each insert is placed into a vial, which is then placed in a humidity cabinet (88% relative humidity at 30°C) for two to four days. After removal from the humidity cabinet, the vials are stoppered and then capped. The vials containing the hydrated insert are then sterilized by means known in the art such as irradiation with high energy electron beams, gamma radiation with a suitable radiation source such as $Co^{60}$.

Illustrative effects obtained by the use of the alkali metal salts of carbonic anhydrase inhibitors appear in the following tables.

TABLE I

EFFECTS OF ACETAZOLAMIDE DIPOTASSIUM SALT, DICHLORPHENAMIDE DIPOTASSIUM SALT AND METHAZOLAMIDE SODIUM SALT TOPICALLY ADMINISTERED ON NORMAL INTRAOCULAR PRESSURE IN UNANESTHETIZED RABBITS

| Compounds ** | Drug Conc. % By Wt. | N | Mean Intraocular Pressure ± SD (KPa) Time (min) after Instillation | | | | | | |
|---|---|---|---|---|---|---|---|---|---|
| | | | 0 | 30 | 60 | 120 | 180 | 240 | 300 |
| Control | | 14 | 2.53 ± 0.17 | 2.53 ± 0.17 | 2.66 ± 0.19 | 2.53 ± 0.21 | 2.53 ± 0.25 | 2.66 ± 0.24 | 2.80 ± 0.23 |
| Acetazolamide Dipotassium salt | 12.1 | 14 | 2.53 ± 0.17 | 2.53 ± 0.17 | 2.66 ± 0.19 | 2.53 ± 0.25 | 2.53 ± 0.25 | 2.66 ± 0.24 | 2.80 ± 0.23 |
| Control | | 14 | 2.66 ± 0.23 | 2.66 ± 0.55 | 2.66 ± 0.47 | 2.53 ± 0.36 | 2.53 ± 0.48 | 2.66 ± 0.42 | 2.92 ± 0.50 |
| Dichlorphenamide Dipotassium salt | 12.1 | 14 | 2.66 ± 0.24 | 2.40 ± 0.32 | 2.40 ± 0.32 | 2.26 ± 0.28 | 1.99 ± 0.32 | 2.26 ± 0.24 | 2.66 ± 0.34 |
| Control | | 14 | 2.80 ± 0.21 | 2.53 ± 0.26 | 2.53 ± 0.30 | 2.66 ± 0.26 | 2.53 ± 0.34 | 2.66 ± 0.20 | 2.80 ± 0.47 |
| Methazolamide Sodium salt | 12.1 | 14 | 2.66 ± 0.25 | 2.66 ± 0.40 | 2.66 ± 0.36 | 2.53 ± 0.44 | 2.40 ± 0.36 | 2.66 ± 0.45 | 2.66 ± 0.37 |

*Significantly different P 0.05 from the control group at corresponding time intervals using student's T test.

**Both eyes of the drug-treated group were instilled with 50μl of the indicated concentration. The central group received 50μl of 0.5% hydroxyethylcellulose in both eyes.

N = number of eyes.

Each salt is administered in 0.5% hydroxyethylcellulose.

TABLE II

EFFECTS OF METHAZOLAMIDE SODIUM SALT IN 0.5% HYDROXYETHYL CELLULOSE TOPICALLY ADMINISTERED ON IOP IN RABBITS WITH ALPHA CHYMOTRYPSIN-INDUCED OCULAR HYPERTENSION**

| Animal No. | Intraocular pressure in (mm Hg) KPa (measured by tonometry) | | | | | | |
|---|---|---|---|---|---|---|---|
| | 0 min | 30 min | 60 min | 120 min | 180 min | 240 min | 300 min |
| 1 | (48) 6.4 | (47) 6.2 | (46) 6.1 | (47) 6.2 | (39) 5.2 | (45) 6.0 | (44) 5.8 |
| 2 | (32) 4.2 | (29) 3.8 | (31) 4.1 | (29) 3.8 | (30) 4.0 | (29) 3.8 | (29) 3.8 |
| 3 | (42) 5.6 | (29) 3.8 | (28) 3.7 | (23) 3.0 | (28) 3.7 | (24) 3.2 | (22) 2.9 |
| 4 | (43) 5.7 | (40) 5.3 | (41) 5.4 | (37) 4.9 | (36) 4.8 | (39) 5.2 | (38) 5.0 |
| 5 | (41) 5.4 | (35) 4.6 | (33) 4.4 | (34) 4.5 | (34) 4.3 | (33) 4.4 | (29) 3.8 |
| 6 | (26) 3.5 | (26) 3.5 | (23) 3.0 | (21) 2.8 | (22) 2.9 | (25) 3.3 | (25) 3.3 |
| 7 | (34) 4.5 | (35) 4.6 | (32) 4.2 | (35) 4.6 | (30) 4.0 | (29) 3.8 | (31) 4.1 |
| 8 | (47) 6.2 | (47) 6.2 | (38) 5.0 | (42) 5.3 | (40) 5.3 | (40) 5.3 | (40) 5.3 |
| + mean | (39.1 ± 7.72)<br>5.20 ± 1.03 | (36.0 ± 8.09)<br>4.79 ± 1.07 | (34.0 ± 7.37)<br>4.52 ± 0.98 | (33.5 ± 8.91)<br>4.46 ± 1.18 | (32.4 ± 6.05)<br>4.31 ± 0.80 | (33.8 ± 7.91)<br>4.50 ± 1.05 | (32.3 ± 7.67)<br>4.30 ± 1.02 |
| ++ mean ΔP | | (−3.1 ± 4.58)<br>−0.41 ± 0.61 | (−5.1 ± 4.68*)<br>−0.67 ± 0.62 | (−5.6 ± 6.02*)<br>−0.74 ± 0.80 | (−6.8 ± 3.70*)<br>−0.90 ± 0.49 | (−5.4 ± 5.42*)<br>−0.72 ± 0.72 | (−6.9 ± 6.27*)<br>−0.92 ± 0.83 |

+ mean ± stand deviation

++ mean of ΔP versus IOP at $t_0$

* significant for $P \leqslant 0.05$ by << + >> paired method

** instilled volume: 50μl

TABLE III

EFFECTS OF ACETAZOLAMIDE DIPOTASSIUM SALT, DICHLORPHENAMIDE DIPOTASSIUM SALT AND METHAZOLAMIDE SODIUM SALT TOPICALLY ADMINISTERED ON INTRAOCULAR PRESSURE (IOP) IN RABBITS WITH ALPHA CHYMOTRYPSIN-INDUCED OCULAR HYPERTENSION

| Compounds ** | Drug Conc. % By Wt. | N[1] | IOP in KPa (mm Hg) t=0 min | Change in IOP KPa (mm Hg) at Various Time (min) Intervals After Drug Administration | | | | | |
|---|---|---|---|---|---|---|---|---|---|
| | | | | 30 | 60 | 120 | 180 | 240 | 300 |
| Acetazolamide Dipotassium Salt | 12.1 | 9 | (37 ± 6.4)<br>4.9 ± 0.8 | (−1 ± 3.1)<br>−0.1 ± 0.4 | (−3 ± 4.8)<br>−0.4 ± 0.6 | (−5 ± 4.6)*<br>−0.6 ± 0.6 | (−6 ± 5.3)*<br>−0.7 ± 0.7 | (−6 ± 5.3)*<br>−0.7 ± 0.7 | (−3 ± 6.5)<br>−0.4 ± 0.8 |
| Dichlorphenamide Dipotassium Salt | 12.1 | 9 | (38 ± 5.9)<br>5.0 ± 0.7 | (−1 ± 5.3)<br>−0.1 ± 0.7 | (−4 ± 3.8)*<br>−0.5 ± 0.5 | (−7 ± 6.2)*<br>−0.9 ± 0.8 | (−6 ± 6.8)*<br>−0.7 ± 0.9 | (−4 ± 6.2)<br>−0.5 ± 0.8 | (−3 ± 6.7)<br>−0.4 ± 0.8 |
| Methazolamide Sodium Salt | 12.1 | 9 | (39 ± 7.7)<br>5.2 ± 1.0 | (−3 ± 4.6)<br>−0.4 ± 0.6 | (−5 ± 4.7)*<br>−0.6 ± 0.6 | (−6 ± 6.0)*<br>−0.7 ± 0.7 | (−7 ± 3.7)*<br>−0.9 ± 0.4 | (−5 ± 5.4)*<br>−0.6 ± 0.5 | (−7 ± 6.3)*<br>−0.9 ± 0.8 |

Results are mean ± srandard deviation.

t = 0 represents the IOP immediately prior the drug administration which served as control.

*P ⩽ 0.05 by << + >> paired method.

[1]N = number of eyes.

**Each salt is administered in 50$\mu$l of 0.5% hydroxyethylcellulose.

The alkali metal salts of carbonic anhydrase inhibitors can be used in combination with each other or with other ophthalmic agents including miotics, epinephrine, timolol, atenolol, propanolol and the like. They are especially useful when combined with these agents since such agents are effective by means of mechanisms other than carbonic anhydrase inhibition. Therefore, the pressure reducing effects are cumulative.

Example 2

Preparation of N-[5-(aminosulfonyl)-3-methyl-1,3,4-thiadiazol-2(3H)-ylidene]acetamide Potassium Salt

To a solution of 0.1*N* potassium hydroxide in methanol (1.19 l, 0.119 mole) is added distilled water (300 ml) and N-[5-(aminosulfonyl)-3-methyl-1,3,4-thiadiazol-2(3H)-ylidene]acetamide (28.12 g, 0.119 mole) at room temperature. The resulting mixture is swirled until complete dissolution of the suspended solid occurs, whereupon, the clear solution is evaporated *in vacuo* at 40°C leaving a colorless residual solid. The solid is dissolved in distilled water (300 ml) providing a clear solution which is evaporated *in vacuo* at 40°C. The residual solid is collected and dried over phosphorous pentoxide at 100°C *in vacuo* for 16 hours to afford analytically pure N-[5-(aminosulfonyl)-3-methyl-1,3,4-thiadiazol-2-(3H)-ylidene]acetamide potassium salt as a colorless solid (30.2 g, 92.6%), m.p. 260°—261°C with dec.

Example 3

Preparation of N-[5-(aminosulfonyl)-3-methyl-1,3,4-thiadiazol-2(3H)-ylidene]acetamide Sodium Salt

To 0.1*N* sodium hydroxide (120 ml, 0.12 mole) is added N-[5-(aminosulfonyl)-3-methyl-1,3,4-thiadiazol-2(3H)-ylidene]acetamide (28.32 g, 0.12 mole) with stirring at room temperature. Stirring is continued at room temperature until a solution is obtained. The resulting solution is diluted with distilled water (120 ml) and filtered. Evaporation of the clear filtrate *in vacuo* at 40°C provides a colorless solid residue which is dried *in vacuo* at 80°C for 16 hours, collected and re-dried *in vacuo* over phosphorous pentoxide at 100°C for four hours to afford analytically pure N-[5-(aminosulfonyl)-3-methyl-1,3,4-thiadiazol-2(3H)-ylidene]acetamide sodium salt as a colorless solid (28.75 g, 92.8%), m.p. 230°—235°C.

Prepared analogously using equivalent molar quantities of reactants are other carbonic anhydrase inhibitor alkali metal salts such as those illustrated in Table IV below.

TABLE IV

CARBONIC ANHYDRASE INHIBITOR SALTS

| Compound | m.p. (°C With Dec.) |
|---|---|
| 4,5-Dichloro-m-benzenedisulfonamide dipotassium salt . hemihydrate | 215—220 |
| 4,5-Dichloro-m-benzenedisolfonamide disodium salt . hemihydrate | >300 |
| 4,5-Dichloro-m-benzenedisulfonamide dirubidium salt | >260 |
| N-[5-(aminosulfonyl)-1,3,4-thiadiazol-2-yl]-acetamide dipotassium salt | 220 |
| N-[5-(aminosulfonyl)-1,3,4-thiadiazol-2-yl]-acetamide disodium salt . 0.25 hydrate | 218—222 |

It has been noted that the sodium salts of carbonic anhydrase inhibitors, when administered in the form of inserts, as opposed to liquid formulation administered by means of drops into the eye, display a more rapid onset of ocular hypotensive activity than the potassium salts of carbonic anhydrase inhibitors. However, these two salt forms (e.g., sodium and potassium), achieve comparable ceiling effects. Therefore, when rapid onset of action is desired it is preferred to employ the sodium salt when inserts are used as the carrier or vehicle.

When using inserts to administer the medication one insert per day per eye is suitable. However, as with all medication, medical monitoring of symptoms must be employed to enable one to arrive at the optimum dosage for the patient.

**Claims for the Contracting States: BE CH DE FR GB IT LU NL SE**

1. An ophthalmic insert for lowering intraocular pressure characterized in that it comprises a mixture of at least:

—a pressure reducing amount of an alkali metal salt of a carbonic anhydrase inhibitor selected among 4,5 - dichloro - m - benzene - disulfonamide; N - [5 - (aminosulfonyl) - 3 - methyl - 1,3,4 - thiadiazol - 2(3H) - ylidene] - acetamide; N - [5 - (aminosulfonyl) - 3 - methyl - 1,3,4 - thiadiazol - 2(3H) - ylidene] - acetamide; p - sulfamoylbenzoic acid; N - [5 - (aminosulfonyl) - 1,3,4 - thiadiazol - 2 - yl]propanamide; N - [5 - (aminosulfonyl) - 1,3,4 - thiadiazol - 2 - yl]butanamide; and 5 - benzenesulfonamido - 1,3,4 - thiadiazol - 2 - sulfonamide; and

—an ophthalmologically acceptable water soluble polymer;

the polymer being shaped under the form of a solid insert suitable to be put in the eye.

2. An insert according to claim 1 where the alkali metal salt of the carbonic anhydrase inhibitor is from 0.1 to 15% by weight of said composition.

3. An insert according to any one of claims 1 and 2 where the salt is potassium or sodium salt.

4. An insert according to any one of claims 1 to 3 where there is included in the composition a preservative so said composition is bacteriostatic.

5. An insert according to any one of claims 1 to 4 which comprises in addition timolol.

**Claims for the Contracting State: AT**

1. A process for preparation of an ophthalmic insert for lowering intraocular pressure characterized in that it comprises

a) mixing at least:

—a pressure reducing amount of an alkali metal salt of a carbonic anhydrase inhibitor selected among 4,5 - dichloro - m - benzene - disulfonamide; N - [5 - (aminosulfonyl) - 3 - methyl - 1,3,4 - thiadiazol - 2(3H) - ylidene] - acetamide; N - [5 - (aminosulfonyl) - 3 - methyl - 1,3,4 - thiadiazol - 2(3H) - ylidene] - acetamide; p - sulfamoylbenzoic acid; N - [5 - (aminosulfonyl) - 1,3,4 - thiadiazol - 2 - yl]propanamide; N - [5 - (aminosulfonyl) - 1,3,4 - thiadiazol - 2 - yl]butanamide; and 5 - benzenesulfonamido - 1,3,4 - thiadiazol - 2 - sulfonamide; and

—an ophthalmologically acceptable water soluble polymer; and

b) shaping the polymer under the form of a solid insert suitable to be put in the eye.

2. A process according to claim 1 where the alkali metal salt of the carbonic anhydrase inhibitor is from 0,1 to 15% by weight of said composition.

3. A process according to any one of claims 1 and 2 where the salt is potassium or sodium salt.

4. A process according to any one of claims 1 to 3 where there is included in the mixture a preservative so said composition is bacteriostatic.

5. A process according to any one of claims 1 to 4 which comprises addition of timolol to the mixture.

**Patententansprüche für die Vertragsstaaten: BE CH DE FR GB IT LU NL SE**

1. Ophthalmische Einlage zur Senkung des intraokularen Drucks, dadurch gekennzeichnet, daß sie ein Gemisch enthält aus mindestens:

einer druckverringernden Menge eines Alkalimetallsalzes eines Carboanhydrase-Inhibitors, ausgewählt aus 4,5 - Dichlor - m - benzol - disulfonamid; N - [5 - (Aminosulfonyl) - 3 - methyl - 1,3,4 - thiadiazol - 2(3H) - yliden] - acetamid; N - [5 - (Aminosulfonyl) - 3 - methyl - 1,3,4 - thiadiazol - 2(3H) - yliden] - acetamid; p - Sulfamoylbenzoesäure; N - [5 - (Aminosulfonyl) - 1,3,4 - thiadiazol - 2 - yl]propanamid; N - [5 - (Aminosulfonyl) - 1,3,4 - thiadiazol - 2 - yl]butanamid; und 5 - Benzolsulfonamido - 1,3,4 - thiadiazol - 2 - sulfonamid; und

ein ophthalmologisch brauchbares wasserlösliches Polymeres;

wobei das Polymere zu einer Form geformt ist, die zum Einsetzen in das Auge geeignet ist.

2. Einlage nach Anspruch 1, in der das Alkalimetallsalz des Carboanhydrase-Inhibitors 0,1 bis 15 Gew.-% der Zusammensetzung beträgt.

3. Einlage nach einem der Ansprüche 1 und 2, in der das Salz Kalium- oder Natriumsalz ist.

4. Einlage nach einem der Ansprüche 1 bis 3, in deren Zusammensetzung ein Konservierungsmittel einbezogen ist, so daß die Zusammensetzung bakteriostatisch ist.

5. Einlage nach einem der Ansprüche 1 bis 4, die zusätzlich Timolol enthält.

**Patentansprüche für den Vertragsstaat: AT**

1. Verfahren zu herstellung ophthalmische Einlage zur Senkung des intraokularen Drucks, dadurch gekennzeichnet, dass man mindestens:

einer druckverringernden Menge eines Alkalimetallsalzes eines Carboanhydrase-Inhibitors, ausgewählt aus 4,5 - Dichlor - m - benzol - disulfonamid; N - [5 - (Aminosulfonyl) - 3 - methyl -

1,3,4 - thiadiazol - 2(3H) - yliden] - acetamid; N - [5 - (Aminosulfonyl) - 3 - methyl - 1,3,4 - thiadiazol - 2(3H) - yliden] - acetamid; p - Sulfamoylbenzoesäure; N - [5 - (Aminosulfonyl) - 1,3,4 - thiadiazol - 2 - yl]propanamid; N - [5 - (Aminosulfonyl) - 1,3,4 - thiadiazol - 2 - yl]butanamid; und 5 - Benzolsulfonamido - 1,3,4 - thiadiazol - 2 - sulfonamid; und

ein ophthalmologisch brauchbares wasserlösliches Polymeres mischt;

und das Polymere zu einer Form geformt ist, die sum Einsetzen in das Auge geeignet ist.

2. Verfahren nach Anspruch 1, in der das Alkalimetall-salz des Carboanhydrase-Inhibitors 0,1 bis 15 Gew.-% der Zusammensetzung beträgt.

3. Verfahren nach einem der Ansprüche 1 und 2, in der das Salz Kalium- oder Natriumsalz ist.

4. Verfahren nach einem der Ansprüche 1 bis 3, in deren Gemisch ein Konservierungsmittel einbezogen ist, so dass die Zusammensetzung Bakteriostatisch ist.

5. Verfahren nach einem der Ansprüche 1 bis 4, in deren Gemisch Timolol ein bezogen ist.

**Revendications pour les etats Contractants: BE CH DE FR GB IT LU NL SE**

1. Insert ophtalmique pour abaisser la tension intraoculaire, caractérisé en ce qu'il comprend un mélange d'au moins:

—une quantité réduisant la tension d'un sel de métal alcalin d'un inhibiteur d'anhydrase carbonique choisi entre le 4,5 - dichloro - m - benzène - disulfonamide; le N - [5 - (aminosulfonyl) - 3 - méthyl - 1,3,4 - thiadiazol - 2(3H) - ylidène] - acétamide; le N - [5 - (aminosulfonyl) - 3 - méthyl - 1,3,4 - thiadiazol - 2(3H) - ylidène] - acétamide; l'acide p-sulfamoylbenzoïque; le N - [5 - (aminosulfonyl) - 1,3,4 - thiadiazol - 2 - yl] - propanamide; le N - [5 - (aminosulfonyl) - 1,3,4 - thiadiazol - 2 - yl]butanamide; et le 5-benzènesulfonamido - 1,3,4 - thiadiazole - 2 - sulfonamide; et

—un polymère soluble dans l'eau ophtalmologiquement acceptable;

le polymère recevant la forme d'un insert soluble approprié pour être mis dans l'oeil.

2. Insert selon la revendication 1 où le sel de métal alcalin de l'inhibiteur d'anhydrase carbonique est à 0,1 à 15% en poids de ladite composition.

3. Insert selon l'une quelconque des revendications 1 et 2 où le sel est le sel de potassium ou de sodium.

4. Insert selon l'une quelconque des revendications 1 à 3 où l'on comprend dans la composition un agent de conservation de manière que ladite composition soit bactériostatique.

5. Insert selon l'une quelconque ces revendications 1 à 4 comprenant en outre du timolol.

**Revendications pour l'etat Contractant: AT**

1. Procédé de préparation d'un insert ophtalmique pour abaisser la tension intraoculaire, caractérisé en ce que:

a) on mélange au moins:

—une quantité réduisant la tension d'un sel de métal alcalin d'un inhibiteur d'anhydrase carbonique choisi entre le 4,5 - dichloro - m - benzène - disulfonamide; le N - [5 - (aminosulfonyl) - 3 - méthyl - 1,3,4 - thiadiazol - 2(3H) - ylidène] - acétamide; le N - [5 - (aminosulfonyl) - 3 - méthyl - 1,3,4 - thiadiazol - 2(3H) - ylidène] - acétamide; l'acide p-sulfamoylbenzoïque; le N - [5 - (aminosulfonyl) - 1,3,4 - thiadiazol - 2 - yl] - propanamide; le N - [5 - (aminosulfonyl) - 1,3,4 - thiadiazol - 2 - yl]butanamide; et le 5-benzènesulfonamido - 1,3,4 - thiadiazole - 2 - sulfonamide; et

—un polymère soluble dans l'eau ophtalmologiquement acceptable;

b) on met le polymère sous la forme d'un insert soluble approprié pour être mis dans l'oeil.

2. Procédé selon la revendication 1 où le sel de métal alcalin de l'inhibiteur d'anhydrase carbonique est à 0,1 à 15% en poids de ladite composition.

3. Procédé selon l'une quelconque des revendications 1 et 2 où le sel est le sel de potassium ou de sodium.

4. Procédé selon l'une quelconque des revendications 1 à 3 où l'on incorpore dans le mélange un agent de conservation de manière que ladite composition soit bactériostatique.

5. Procédé selon l'une quelconque des revendications 1 à 4 comprenant en outre l'ajout de timolol au mélange.